# EUROPEAN PATENT SPECIFICATION

(11) **EP 1 436 217 B1**
(45) Date of publication and mention of the grant of the patent: **06.09.2006**
(21) Application number: 02785182.3
(22) Date of filing: 09.10.2002
(51) Int. Cl.: B65D 83/04, A61M 15/00

(54) **MEDICAMENT DISPENSER**
ABGABEVORRICHTUNG FÜR ARZNEIMITTEL
DISTRIBUTEUR DE MEDICAMENTS

(30) Priority: 19.10.2001 GB 0125132
(43) Date of publication of application: 14.07.2004
(73) Proprietor: GLAXO GROUP LIMITED, Greenford, Middlesex UB6 0NN (GB)
(72) Inventor: CROSBY, Gary Thomas, Ware, Herts UB6 0NN (GB); PALMER, Mark Gregory, Ware, Herts UB6 0NN (GB)
(74) Representative: Reeves, Julie Frances
(86) International application number: PCT/EP2002/011315
(87) International publication number: WO 2003/035509

(56) References cited:
- WO-A-01/41846
- WO-A-02/36188
- GB-A- 2 242 134
- US-A- 5 582 162

## Description

### Technical field

The present invention relates to a medicament dispenser for dispensing medicament according to the preamble of claim 1. The invention particularly relates to a device for use in dispensing medicament in powder or tablet form.

### Background to the invention

The use of inhalation devices in the administration of medicaments, for example in bronchodilation therapy is well known. Such devices generally comprise a body or housing within which a medicament carrier is located. Known inhalation devices include those in which the medicament carrier is a blister strip containing a number of discrete doses of powdered medicament. Such devices usually contain a mechanism of accessing these doses, usually comprising either piercing means or means to peel a lid sheet away from a base sheet. The powdered medicament can then be accessed and inhaled. Such a mechanism may also be used for dispensing medicament in tablet form wherein peeling away the lid sheet from the base sheet reveals a tablet for removal and subsequent consumption.

It is an object of the present invention to simplify the internal mechanism of a medicament dispenser for dispensing medicament in powder or solid form from a medicament carrier as described *supra.*

Yet another object of the present invention is to provide a device which is refillable by insertion of a replacement cassette containing a medicament carrier. The cassette may be replaced when the medicament carrier is empty. The device is therefore more 'environmentally friendly' as the majority of the device may be retained and is not disposable. It also allows the device to be fitted with additional features such as electronics which may not be cost effective on a completely disposable device.

It is a further object of the present invention that the cassette may be easily removed and that a new replacement cassette can be easily inserted. It is also desirable that the operation of the medicament dispenser be straightforward and non-complex and in particular that the number of separate steps involved in preparing the device for use be minimised. This is especially relevant where the device is designed for use in the delivery of medicament in emergency or rescue situations (e.g. asthma attacks) where simplicity and ease of use is paramount.

When not in use it is desirable from a hygiene standpoint that a mouthpiece, or other medicament exit channel, is provided with some kind of protective cover. The cover desirably acts both to prevent build-up of dirt and to prevent ingress of dirt into, the body of the device through the mouthpiece or channel, which might then be subject to inhalation or consumption by a patient. It is also desirable that the cover is in some way attached or mounted to the device to minimise the risk that the cover is misplaced or lost. It is therefore a further object of the present invention for the body of the device to act as a mouthpiece or exit channel cover when the device is in storage and that the cassette is movable relative to the body to enable the mouthpiece or channel to be uncovered for use by the patient.

It is a further object of the invention to provide a medicament dispenser device suitable for use with a large number of discrete doses but which is of an acceptable size for use by patients.

In GB-A-2 242 134 there is disclosed a medicament dispenser having the features of the pre-characterising part of claim 1.

### Summary of the invention

Accordingly, in one aspect the invention provides medicament dispenser according to claim 1.

The medicament dispenser herein is suitable for use with a medicament carrier having a plurality of pockets for containing medicament wherein said pockets are essentially uniformly spaced along the length of and defined between two peelably separable sheets secured to each other. The medicament carrier is generally in the form of an elongate, peelable blister strip.

As used herein, the term wheel encompasses, for example, a wheel, spindle or spool. The wheel itself has a fixed diameter, although its effective winding diameter (i.e. wheel plus thickness of lid sheet wound therearound) will vary in use as lid sheet is accommodated thereby. The wheel is typically of solid construction, and essentially incompressible in nature, at least about its diameter.

A problem encountered with the use of such a wheel as the lid-driving means for driving a lid sheet of a medicament carrier is that as the lid sheet winds up around the wheel the effective winding diameter of the wheel increases, and therefore its effective lateral pulling action (i.e. length of pull) also increases. This is problematic because it is desirable that on actuation, a definable pull action is experienced by the medicament carrier pocket at the opening station to ensure that a generally uniform indexing/opening effect is experienced by each pocket of the medicament carrier. In general terms, insufficient pull action will result in failure to open up the pocket whilst excess pull will stress mechanical components and increase the force required to actuate the dispenser.

One solution to the above problem could involve the use of a wheel of variable actual diameter, for example a wheel of a collapsible nature whose actual diameter reduces as lid sheet is wound therearound. Such wheel forms can however, be more difficult to design/manufacture than a wheel of fixed diameter whose form is non-collapsible.

In accord with the present invention, compensating means as set forth in claim 1 are provided to compensate for any increase in the diameter of the effective winding surface of the wheel during use of the dispenser and thereby to ensure that said medicament carrier is uniformly indexed upon each actuation of said dispensing mechanism.

Suitably, the wheel takes a hub form and the torsion spring is accommodated such as to provide a torsion hub drive; which may be driven by gears. In one aspect, in use, the torsion spring is initially tense and the tension reduces as the wheel receives lid sheet thereby also reducing its drive action on later-received lid sheet (i.e. that lid sheet towards the non-already received end of the medicament carrier).

It will have been appreciated that the compensating means functions such as to compensate for an increase in the diameter of the effective winding surface of the wheel during use of the dispenser. It will be appreciated that the initial effective winding surface and associated initial drive 'speed' of the wheel is principally a function of the (fixed) initial diameter of the wheel. Variations are envisaged herein where that initial effective winding surface is selected to define particularly selected initial drive characteristics of the wheel.

In one variation sometimes called 'one way take up' mode, the initial effective winding surface is selected such as to initially provide ideal (i.e. uniform) indexing of the medicament carrier. As lid sheet winds up around the-wheel the effective winding surface increases and the compensating means acts such as to compensate for that increase.

In another variation sometimes called 'two way take up' mode, the initial effective winding surface is selected such as to initially provide non-ideal (i.e. non-uniform) indexing of the medicament carrier because the diameter of the wheel is insufficiently great. As lid sheet winds up around the wheel the effective winding surface increases to an ideal diameter and then on further winding up continues to increase to a non-ideal (i.e. too great diameter). In this embodiment it will be appreciated that the degree and nature of compensation provided by the compensating means will vary over the winding up function. The compensating means initially acts such as to compensate for the insufficient wheel diameter. That compensation then decreases to zero at the point where the diameter of the effective winding surface is ideal. The compensation then progressively acts such as to compensate for a too great effective winding surface. This approach has the advantage of overall reducing the (average) compensating action (e.g. tension) experienced by the medicament carrier from a defined zero (i.e. the ideal) and enables the use of less powerful tensioning means (e.g. smaller springs). In a preferred aspect of this variation, the ideal effective winding surface diameter is selected to correspond approximately to the point at which half of the lid sheet is wound up on the wheel, in which case the average (i.e. mean) compensating action experienced is by the carrier over a full usage cycle is close to zero.

Suitably, said indexing means comprises a rotatable index wheel having recesses therein, said index wheel being engageable with a medicament carrier in use with said medicament dispenser such that said recesses each receive a respective pocket of the base sheet of a medicament carrier in use with said medicament dispenser.

Alternatively, said index means may comprise an indexing ratchet which is moveable between a locked position whereby said ratchet engages a pocket on said medicament carrier and prevents further peeling thereof, and a release position allowing free movement of said medicament carrier, and actuation of said medicament dispenser actuates said lid driving means and releases said index ratchet from said medicament carrier to allow peeling thereof.

Suitably, the medicament dispenser further comprises an indexing lever for actuating said dispenser wherein said indexing lever is interconnected with said indexing means and/or said lid driving means.

In one aspect, the lid driving means and/or the indexing means are operated by an electronic drive system. The electronic drive system may also be used in conjunction with a mechanical drive system.

The electronic drive typically comprises a motor, preferably an electrically-powered motor. The motor may provide linear or rotary drive, but in general, rotary motors are most suitable. The motor may for example, comprise a DC electric motor, a piezoelectric (PZ) motor, an ultrasonic motor, a solenoid motor or a linear motor. Preferably, the electronic drive system comprises a DC motor, a PZ motor or an ultrasonic motor.

The use of ultrasonic motors is particularly preferred since they offer advantages over conventional motors in terms of weight, size, noise, cost and torque generated. Ultrasonic motors are well known in the art and are commercially available (e.g. BMSTU Technological Cooperation Centre Ltd, Moscow, Russia; Shinsei Corporation, Tokyo, Japan).

Ultrasonic motors do not use coils or magnets but comprise a piezo-electric ceramic stator which drives a coupled rotor. The stator generates ultrasonic vibrations which in turn causes rotation of the rotor. While regular DC motors are characterised by high speed and low torque, requiring reduction gearing to increase torque, ultrasonic motors attain low speed and high torque, thus eliminating the need for reduction gearing. Furthermore, these motors are lightweight and compact, lacking coils and magnets, and are noiseless as the ultrasonic frequencies used are not audible to the human ear. Suitably, the dispenser further comprises actuating means for actuating said electronic drive system. Said actuating means may take the form of a switch, pushbutton, or lever.

In one aspect, the coil comprising the unused medicament strip is surrounded by a constant force spring. Alternatively the coil comprising the unused medicament strip may be surrounded by an elastomeric band or band comprising a contractible material. The constant force spring, elastomeric band or band comprising a contractible material contracts as the coil reduces in size.

Suitably, said peeling means additionally comprise a guide for guiding the lid sheet and base sheet along separate paths at the opening station. The lid sheet is passed around the guide portion onto the lid driving means.

Alternatively, the guide comprises a roller mechanism. The lid sheet is fed over the rollers onto the lid driving means.

Suitably, the internal mechanism additionally comprises a first chamber in.which the strip is initially housed and from which it is dispensed and a second chamber to receive the used portion of the base sheet after it has been indexed and separated from the lid sheet.

Suitably, said first chamber and said second chamber are separated by a wall.

Suitably, said wall is movable to adjust the size of said first and second chambers.

Alternatively, said wall is flexibly movable to adjust the size of the first and second chambers.

Alternatively, the second chamber is expandable to create space for the growing coil of the used portion of the base sheet.

Suitably, the internal mechanism further comprises a third chamber to receive the used portion of the lid sheet and a fourth chamber which houses the indexing means. The fourth chamber may communicate via a slit, which in turn extends upwardly within a mouthpiece or exit channel and communicates with air inlets.

Suitably, the internal mechanism additionally comprises a crushing wheel to crush the medicament pockets after the medicament has been removed from them. The crushing wheel therefore reduces the space, which the used portion of the base sheet takes up.

Typically, the internal mechanism for accessing said medicament contained within said medicament carrier is housed within a cassette.

In one aspect, the invention provides a medicament dispenser for dispensing medicament comprising: a body; a holder, shaped to fit within said body and movable relative to said body; and receivable by said holder, said cassette containing said medicament carrier.

Suitably, movement of the holder relative to the body results in movement of the cassette between a first position and a second position such that the cassette is reversibly removable from the holder when the cassette is in the second position.

Suitably, the first position comprises a dispensing position. Preferably the second position comprises a non-dispensing position. The cassette is therefore only removable from the holder when the cassette is in the non-dispensing position.

Suitably, the holder and body include attaching means to attach the holder to the body. Preferably said attaching means comprise a snap fit mechanism.

Suitably, said snap fit mechanism comprises a pin and hole system.

Suitably, the holder is pivotally movable relative to the body.

Alternatively, the holder is rotationally movable relative to the body.

Suitably, the holder additionally comprises a stop to limit movement of the holder relative to the body. The stop abuts against the edge of the body at two points when it is rotated. At these points the holder may be designed to click into place. Therefore when the stop abuts one body edge then it is clicked into the dispensing position and when the stop abuts the other body edge then it is clicked into the non-dispensing position.

Suitably, the holder is slidably movable relative to the body.

Suitably, the holder additionally comprises a catch to retain the cassette. The catch may for example comprise a sprung pin which fits into a hole or an integral catch which deforms when pressed allowing removal of the cassette.

Suitably, the catch is child resistant. Child resistance may be realised by having a system which forces the user to perform two actions at once to remove the cassette. Other features of the catch may include shock or impact resistance, the ability to lock the catch and orientation features to ensure that the cassette can only be inserted one way. The catch should also be easy to manufacture and assemble, be robust, be composed of a minimal number of components and intrude minimally into the space into which the cassette is inserted.

Suitably, the holder includes guide means to guide the cassette into the holder. Preferably said guide means comprise ,guide rails. Alternatively the guide means comprise grooves, indentations or other shaping or surface details to define a lock and key' relationship between the holder and the cassette. Colour guides, arrows and any other surface markings may also be employed.

Suitably, the cassette additionally comprises an indexing lever. The indexing lever has a finger tab located outside the body of the cassette. The rest of the indexing lever is located within the cassette. The indexing lever may, have teeth at its tail end and/or teeth along its mid portion.

Suitably, the cassette additionally comprises a mouthpiece.

In one aspect, said mouthpiece is extendable. The mouthpiece extends as the cassette and holder are moved from the non-dispensing position to the dispensing position.

Alternatively, the mouthpiece is retractable. The mouthpiece retracts as the cassette and holder are moved from the dispensing position to the non-dispensing position.

In one aspect, the mouthpiece is telescopic.

In another aspect, the mouthpiece is fixed.

The medicament dispenser may also be designed for nasal inhalation of a powdered medicament and may therefore incorporate a nosepiece as an alternative to a mouthpiece. If fhe medicament is in solid form, the dispenser may incorporate an exit channel for tablet release.

Suitably; the body covers the mouthpiece and indexing lever when the cassette is in the non-dispensing position. This avoids the need for a separate cover and protects the mouthpiece from the ingress of dirt and contaminants during storage.

Suitably, the cassette additionally comprises a raised portion to fit against the holder. The raised portion is located at the opposite end of the cassette to the mouthpiece/nosepiece/exit and indexing lever and prevents the incorrect insertion of the cassette into the holder since it is too wide to fit into the holder. The raised portion is shaped such that it fits against a cut away part of the holder. Preferably said raised portion includes a section which is raised to define a grip portion.

Suitably, at least a portion of the holder and body are shaped for ease of grip by the user.

Suitably, operation of the device may be performed with one hand.

Suitably, the body additionally comprises at least one brush or wiper blade located along its top or bottom side which brush against the top and bottom surfaces of the inside of the cassette. The brush or wiper blade acts to close off the chamber for enclosing the medicament carrier from the rest of the body of the cassette and to prevent any loose powder from entering the rest of the cassette. Loose powder may enter the chamber from the used portion of the blister strip if the patient indexes the strip by pressing the lever when they do not intend to take a dose or when they fail to inhale all the powder.

Suitably, the medicament dispenser comprises an actuation or dose counter for counting the number of actuations of the indexing lever or releases of dose from the cassette. The dose counter may count the number of doses left to be taken or the number of doses taken.

Suitably, said dose counter is electronic. Alternatively, said dose counter is mechanical.

Suitably, said dose counter is located within the cassette. Alternatively, the dose counter is external to the cassette.

Alternatively, the blister strip has printed numbers on it corresponding to the doses in the pockets. Preferably said printed numbers are visible through a window in the cassette. The device may be assembled as follows. The holder is snap fitted into the body. The cassette is assembled separately. The body of the cassette is formed, preferably in two sections with any necessary spindles or integral components formed into the base. Individual components such as indexing wheels, lid winding mechanisms, guide portions etc are then assembled into the base. Finally the medicament containing blister strip (or other suitable medicament carrier) may be inserted into the cassette. This may be wound into the device before the lid is attached to the cassette and the cassette sealed. Alternatively, the cassette may be formed completely apart from a hole left in its side for insertion of the blister strip or medicament carrier. The hole may then be sealed to complete the cassette. This second method of inserting the medicament carrier into the device has the advantage that it is much simpler.

The medicament may comprise a capsule, pellet or tablet. Alternatively, the medicament may be in powdered form. Preferably, when in powdered form the medicament comprises a drug. Preferably the drug is selected from the group consisting of albuterol, salmeterol, fluticasone propionate and beclomethasone dipropionate and salts or solvates thereof and any combination thereof. Preferably said combination comprises salmeterol xinafoate and fluticasone propionate.

Suitably, the powdered medicament additionally comprises an excipient. Suitably, said excipient is a sugar.

### Brief Description of the Drawings

The invention will now be described with reference to the accompanying drawings in which:
Figure 1 shows a perspective view of a medicament carrier in accordance with the present invention;
Figure 2 shows a base unit housing an internal mechanism not in accordance with the invention;
Figure 3 shows a base unit housing an internal mechanism not according to the invention;
Figure 4 shows a perspective view of a medicament dispenser not according to the invention with the cassette removed from the holder and the body;
Figure 5 shows a base unit housing an internal mechanism according to an aspect of the invention; and
Figure 6 shows a graphic representation of strip lid foil tension versus strip pocket number for 'one way take up' and 'two way take up' modes herein.

### Detailed Description of the Drawings

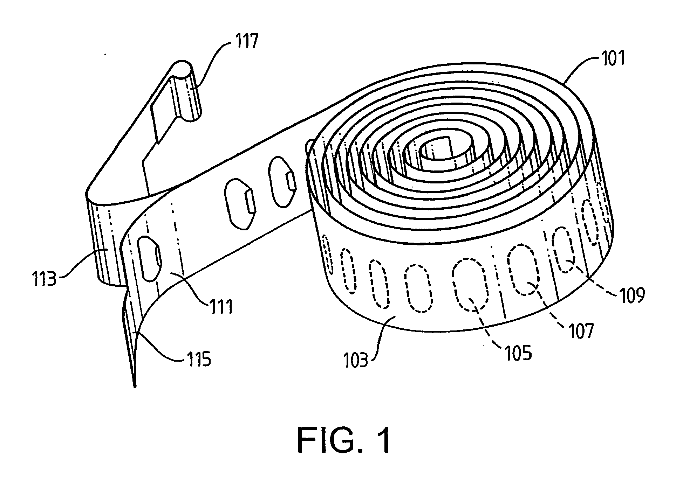

Referring now to the Figures, Figure 1 shows a medicament carrier 101 in accord with the present invention. The medicament carrier comprises a flexible strip 103 defining a plurality of pockets 105, 107, 109 each of which contains a dose of medicament which can be inhaled, in the form of powder.

The strip comprises a base sheet 111 in which blisters are formed to define the pockets. 105, 107, 109 and a lid sheet 113 which is hermetically sealed to the base sheet except in the region of the blisters in such a manner that the lid sheet 113 and the base sheet 111 can be peeled apart. The sheets 111, 113 are sealed to one another over their whole width except for the leading end portions 115, 117 where they are preferably not sealed to one another at all. The lid 113 and base 111 sheets are each preferably formed of a plastics/aluminium laminate and are preferably adhered to one another by heat sealing.

The strip 103 is shown as having uniformly-spaced elongate pockets 105, 107, 109 that run transversely with respect to the length of the strip 103. This is convenient in that it enables a large number of pockets 105, 107, 109 to be provided in a given strip 103 length. The strip 103 may, for example, be provided with sixty or one hundred pockets but it will be understood that the strip 103 may have any suitable number of pockets.

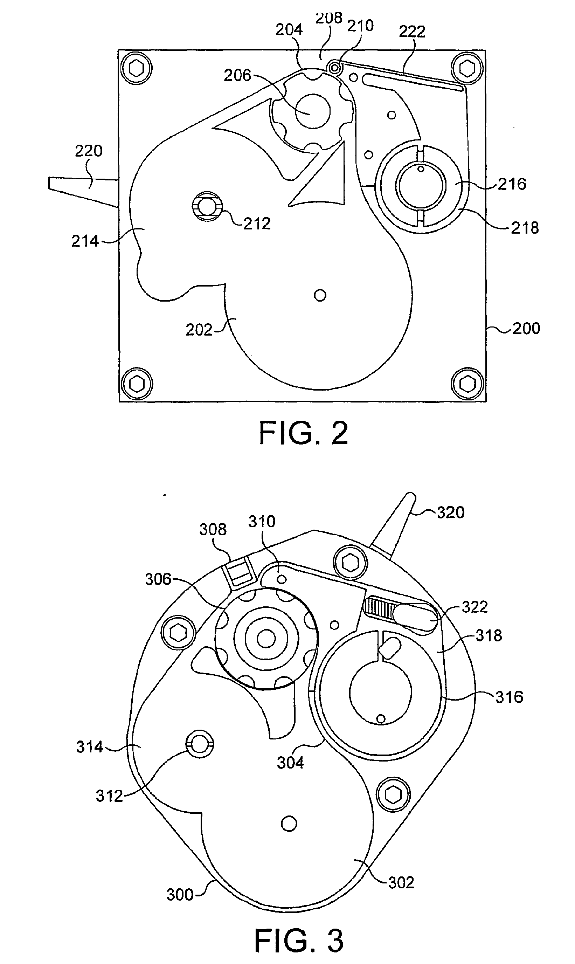

Figure 2 illustrates a base unit 200 of a first medicament dispenser. A medicament strip (not shown for clarity) is positioned in chamber 202 of the base unit 200. The strip is pre-fed through a guide member 204 within the manifold component and engaged in a six-pocket index wheel 206. The first pocket of the strip is positioned one pocket away from the opening station 208. The lid foil and base foil are separable about a beak 210. The resulting empty base foil is coiled about a base take-up spindle 212 in the base take-up chamber 214. The used lid foil is fed over the beak 210 and coiled about a lid take-up spindle 216 in the lid take-up chamber 218.

To actuate the dispenser, the index lever 220 is turned until it reaches a stop (not shown). The index lever 220 is interconnected with the index wheel 206, the base take-up spindle 212 and the lid take-up spindle 216, through a simple gear train (not shown). The index lever 220 thus indexes the strip and coils up the waste foils.

Initially, the gearing between the index wheel 206 and the lid take-up foil spindle 216 is one-to-one. However, as the lid take up spindle 216 winds on more foil, its effective winding diameter increases. An increase in diameter would cause the lid take-up spindle 216 to pull more strip than the index lever 220 releases. Thus, compensating means in the form of a flexible member 222, which is positioned in the lid foil path between the index wheel 206 and the lid take-up spindle 216, flexes, such that the flexible member 222 reduces the distance between the index wheel 206 and the lid take-up spindle 216 and thereby compensates for the incremental change in strip length pulled by the take-up spindle 216.

Figure 3 illustrates a base unit 300 of a second medicament dispenser. A medicament strip (not shown for clarity) is positioned in chamber 302 of the base Unit 300. The strip is pre-fed through a guide member 304 within the manifold component and engaged in eight-pocket index wheel 306. The first pocket of the strip is positioned one pocket away from the opening station 308. The lid foil and base foil are separable about a beak 310. The resulting empty base foil is coiled about a base take-up spindle 312 in the base take-up chamber 314. The used lid foil is fed over the beak 310 and coiled about a lid take-up spindle 316 in the lid take-up chamber 318.

To actuate the dispenser, the index lever 320 is turned until it reaches a stop (not shown). The index lever 320 is interconnected with the index wheel 306, the base take-up spindle 312 and the lid take-up spindle 316, through a simple gear train (not shown). The index lever 320 thus indexes the strip and coils up the waste foils.

Initially, the gearing between the index wheel 306 and the lid take-up foil spindle 316 is one-to-one. However, as the lid take up spindle 316 winds on more foil, its effective winding diameter increases. An increase in diameter would cause the lid take-up spindle 316 to pull more strip than the index lever 320 releases. Thus, compensating means in the form of a sprung-loaded tensioner 322, which is positioned in the lid foil path between the index wheel 306 and the lid take-up spindle 316, flexes, such that the sprung-loaded tensioner 322 reduces the distance between the index wheel 306 and the lid take-up spindle 316 and thereby compensates for-the incremental change in strip length pulled by the take-up spindle 316.

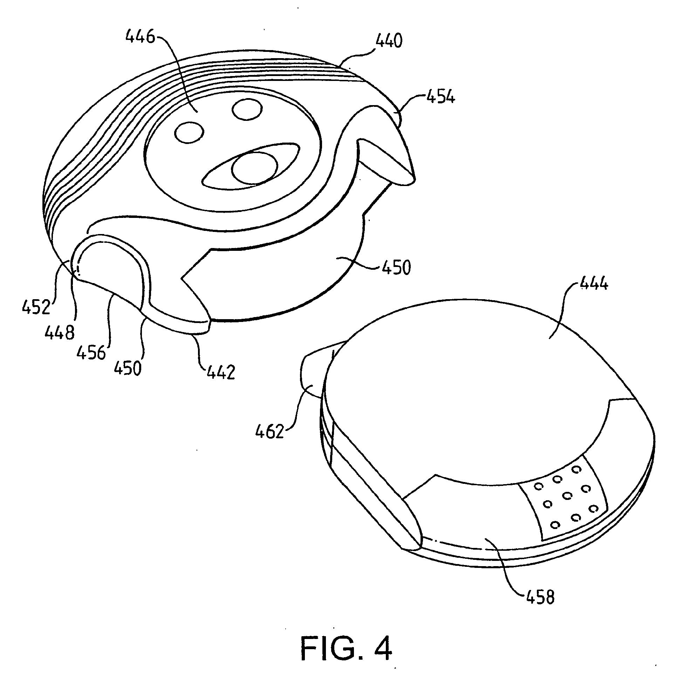

Figure 4 shows a third medicament dispenser comprising a body 440, a holder 442, refill cassette 444 and electronic display 446. The holder 442 is shaped to fit snugly inside body 440 and is fixed to a point on the body (not shown) about which it rotates. Stops 448, 450 protrude from the holder 442 and prevent the holder 442 from rotating more than about 180° relative to the body 440. The stops 448, 450 also provide two defined positions of the holder 442 within the body 440. One position is defined by stop 448 meeting with body edge 452 and the other position defined by stop 450 meeting with body edge 454 when the holder has been rotated relative to the body. The area between stops 448 and 450 is shaped to form a thumb or finger grip 456 for the user of the device. The holder 442 forms a shell into which the refill cassette 444 snugly fits.

The refill cassette 444 comprises a shell containing the medicament carrier (not shown) and a mechanism for opening the carrier (not shown) for the medicament to be accessed. The refill cassette 444 has a raised portion 458 at one end on both sides along its width so that this part of the refill cassette 444 is at least the same depth as the part of the holder 460 which receives the refill cassette 444. This allows the position of the cassette 444 within the holder 442 to be fixed such that the ridge 458 protrudes from the holder 442 but the rest of the cassette 444 is contained within the holder 442.

The refill cassette 444 also has a mouthpiece (not shown) and an indexing lever 462 for indexing the medicament carrier within the cassette 444.

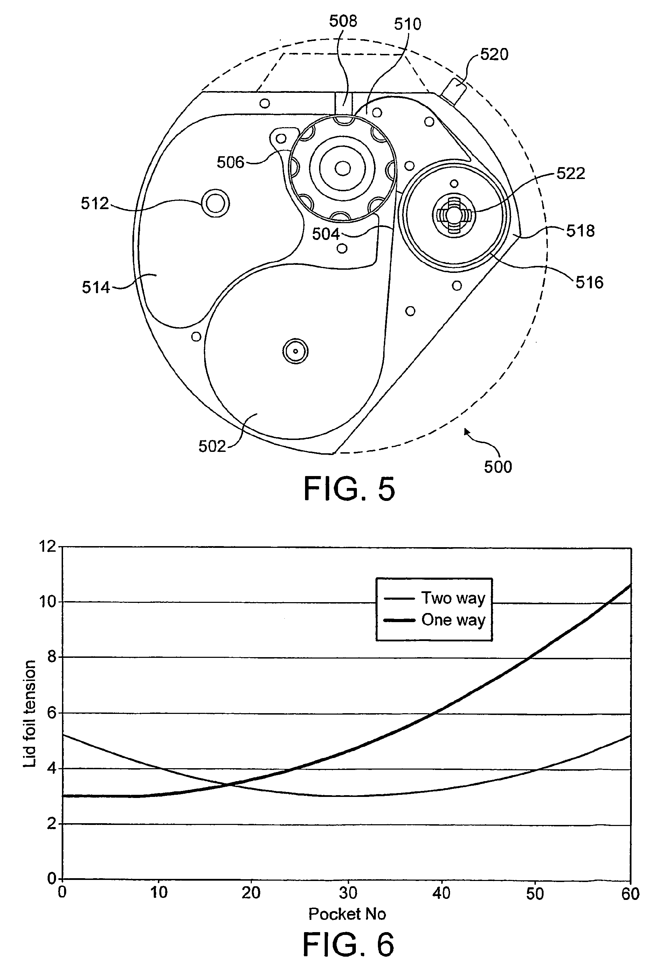

Figure 5 illustrates a base cassette unit 500 of a medicament dispenser according to the invention. A medicament strip (not shown for clarity) is positioned in chamber 502 of the base unit 500. The strip is pre-fed through a guide member 504 within the manifold component and engaged in eight-pocket index wheel 506. The first pocket of the-strip is positioned one pocket away from the opening station 508. The lid foil and base foil are separable about a beak 510. The resulting empty base foil is coiled about a base take-up spindle 512 in the base take-up chamber 514. Used lid foil is feeds over the beak 510 and is coiled about hub-form lid take-up spindle 516 in the lid take-up chamber 518.

To actuate the dispenser, the index lever 520 is turned until it reaches a stop (not shown). The index lever 520 is interconnected with the index wheel 506, the base take-up spindle 512 and the lid take-up spindle 516, through a simple gear train (not visible). The index lever 520 thus indexes the strip and coils up the used foils.

Initially, the gearing between the index wheel 506 and the lid take-up foil spindle 516 is about one-to-one. However, as the lid take up spindle 516 winds on more foil, its effective winding diameter increases. An increase in diameter would cause the lid take-up spindle 516 to pull more strip than the index lever 520 releases. Thus, compensating means in the form of a torsion spring 522, which is positioned in the hub-form lid take-up spindle 516 are provided.

In a first 'one way take up' mode of action, the torsion spring 522 is initially tense and the tension thereof reduces as the lid take-up spindle 516 receives lid sheet thereby gradually reducing its drive action as more lid sheet is received thereon. Compensation is thereby provided for the incremental change in effective winding surface diameter of the take-up spindle 516.

In a variation of the dispenser of Figure 5, the diameter of the hub-form take-up spindle 516 (and hence, its initial effective winding surface diameter) is reduced from that illustrated, and in particular reduced such as to be too small to initially provide uniform (one-to-one) indexing of the medicament carrier. This variation is designed for operation in the 'two way take up' mode. In use, as lid sheet winds up around the lid take-up spindle 516 its effective winding surface increases, ultimately to an ideal diameter for uniform indexing of the medicament carrier. However, on further winding up the diameter of the effective winding surface continues to increase to an excessively large diameter. In this mode of usage the torsion spring 522 initially acts such as to compensate for the insufficient lid take-up spindle diameter. That compensation then decreases to zero at the point where the diameter of the effective winding surface is ideal. The compensation then progressively acts such as to compensate for a too great diameter of effective winding surface.

The 'one way take up' and 'two way take up' modes of action may be better understood by reference to Figure 6. This shows a graphical representation of the tension experienced (y-axis) by the lid foil of an elongate blister strip-form medicament carrier (e.g. as shown in Figure 1) versus pocket number of the medicament carrier (x-axis). The pocket number value may be appreciated to relate directly to how much lid foil has been received by a wheel (e.g. take-up spindle 516 of Figure 5) of the dispenser. The data presented in Figure 6 relates to a sixty pocket medicament carrier blister strip, thus pocket no. 30 approximately represents the mid-strip point.

As may be seen in Figure 6, for the 'one way take up' mode the lid foil tension is initially moderate and gradually increases as the pocket number increases (i.e. as lid foil is wound up about the wheel) to a much higher value. For the 'two way take up' mode the tension is initially moderately high and it very gradually decreases to a minimum at about the mid-strip point (pocket no. 30) before increasing again to a moderately high value at the end-strip point (pocket no. 60). It may be appreciated that the maximum lid foil tension experienced in the 'two way take up' mode is less than that for the 'one way take up' mode. Thus, in general smaller, less involved and more compact compensating means may be employed for the 'two way take up' mode embodiments. It may also be appreciated that a more uniform lid foil tension is experienced in the 'two way take up' mode which can be beneficial from a structural stability standpoint.

Any or all of the dispensers shown in Figures 2, 3 and 5 may be adapted for operation in the 'two way take up' mode by appropriate selection of wheel diameter.

It may be appreciated that any of the parts of the dispenser or cassette which contact the medicament suspension may be coated with materials such as fluoropolymer materials (e.g. PTFE or FEP) which reduce the tendency of medicament to adhere thereto. Any movable parts may also have coatings applied thereto which enhance their desired movement characteristics. Frictional coatings may therefore be applied to enhance frictional contact and lubricants (e.g. silicone oil) used to reduce frictional contact as necessary.

The medicament dispenser of the invention is suitable for dispensing medicament, particularly for the treatment of respiratory disorders such as asthma and chronic obstructive pulmonary disease (COPD), bronchitis and chest infections. Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate (e.g. as the sodium salt), ketotifen or nedocromil (e.g. as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (e.g. as the dipropionate ester), fluticasone (e.g. as the propionate ester), flunisolide, budesonide, rofleponide, mometasone e.g. as the furoate ester), ciclesonide, triamcinolone (e.g. as the acetonide) or 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester; antitussives, e.g., noscapine; bronchodilators, e.g., albuterol (e.g. as free base or sulphate), salmeterol (e.g. as xinafoate), ephedrine, adrenaline, fenoterol (e.g. as hydrobromide), formoterol (e.g. as fumarate), isoprenaline, metaproterenol, phenylephrine, phenylpropanolamine, pirbuterol (e.g. as acetate), reproterol (e.g. as hydrochloride), rimiterol, terbutaline (e.g. as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; adenosine 2a agonists, e.g. 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate); α₄ integrin inhibitors e.g. (2S)-3-[4-({[4-(aminocarbonyl)-1-piperidinyl]carbonyl}oxy)phenyl]-2-[((2S)-4-methyl-2-{[2-(2-methylphenoxy) acetyl]amino}pentanoyl)amino] propanoic acid (e.g. as free acid or potassium salt), diuretics, e.g., amiloride; anticholinergics, e.g., ipratropium (e.g. as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; vaccines, diagnostics, and gene therapies. It will be dear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Preferred medicaments are selected from albuterol, salmeterol, fluticasone propionate and beclomethasone dipropionate and salts or solvates thereof, e.g., the sulphate of albuterol and the xinafoate of salmeterol. Medicaments can also be delivered in combinations. Preferred formulations containing combinations of active ingredients contain salbutamol (e.g., as the free base or the sulphate salt) or salmeterol (e.g., as the xinafoate salt) or formoterol (eg as the fumarate salt) in combination with an anti-inflammatory steroid such as a beclomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt).

Generally, powdered medicament particles suitable for delivery to the bronchial or alveolar region of the lung have an aerodynamic diameter of less than 10 micrometers, preferably less than 6 micrometers. Other sized particles may be used if delivery to other portions of the respiratory tract is desired, such as the nasal cavity, mouth or throat. The medicament may be delivered as pure drug, but more appropriately, it is preferred that medicaments are delivered together with excipients (carriers) which are suitable for inhalation. Suitable excipients include organic excipients such as polysaccharides (i.e. starch, cellulose and the like), lactose, glucose, mannitol, amino acids, and maltodextrins, and inorganic excipients such as calcium carbonate or sodium chloride. Lactose is a preferred excipient.

Particles of the powdered medicament and/or excipient may be produced by conventional techniques, for example by micronisation, milling or sieving. Additionally, medicament and/or excipient powders may be engineered with particular densities, size ranges, or characteristics. Particles may comprise active agents, surfactants, wall forming materials, or other components considered desirable by those of ordinary skill.

The excipient may be included with the medicament via well known methods, such as by admixing, co-precipitating and the like. Blends of excipients and drugs are typically formulated to allow the precise metering and dispersion of the blend into doses. A standard blend, for example, contains 13000 micrograms lactose mixed with 50 micrograms drug, yielding an excipient to drug ratio of 260:1. Dosage blends with excipient to drug ratios of from 1000:1 to 1:1 may be used. At very low ratios of excipient to drug, however, the drug dose reproducibility may become more variable.

It will be understood that the Present disclosure is for the purpose of illustration only and the invention extends to modifications, variations and improvements thereto within the scope of the appended claims.

## Claims

1. A medicament dispenser for use with a medicament carrier (101) having a plurality of pockets (105, 107, 109) for containing medicament wherein said pockets are spaced along the length of and defined between two peelable sheets (111, 113) secured to each other, said dispenser having an internal dispensing mechanism for accessing said medicament contained within said medicament carrier, said mechanism comprising,
a) an opening station (508) for receiving a pocket of said medicament carrier;
b) peeling means positioned to engage a base sheet (111) and a lid sheet (113) of a pocket which has been received in said opening station (508) for peeling apart such a base sheet (111) and lid sheet (113), to open such a pocket, said peeling means including lid driving means for pulling apart a lid sheet (113) and a base sheet (111) of a pocket that has been received at said opening station (508), said lid driving means comprising a fixed-diameter wheel (516) on which said lid sheet (113) is wound, said wheel (516) having an effective winding surface, the diameter of which increases as more lid sheet is wound about said wheel (516),
c) an outlet, positioned to be in communication with an opened pocket through which a user can remove medicament from such an opened pocket; and
d) indexing means (506) for indexing in communication with said outlet, pockets of a medicament carrier in use with said medicament dispenser, said indexing means being interconnected with said lid driving means such that movement of one correlates with the movement of the other,
additionally comprising compensating means to compensate for any increase in the diameter of said effective winding surface during use of the dispenser and to thereby ensure that said medicament carrier is uniformly indexed upon each actuation of said dispensing mechanism **characterised in that** the compensating means comprises a torsion spring (522) positioned at the wheel (516).

2. A medicament dispenser according to claim 1, wherein the wheel (516) has a hub form shaped such as to accommodate said torsion spring (522).

3. A medicament dispenser according to any of claims 1 to 2, wherein the initial effective winding surface is selected such as to initially provide uniform indexing of the medicament carrier.

4. A medicament dispenser according to any of claims 1 to 2, wherein the initial effective winding surface is selected such as to initially provide non-uniform indexing of the medicament carrier.

5. A medicament dispenser according to claim 4, wherein the diameter of the initial effective winding surface is smaller than that required for uniform indexing of the medicament carrier and in use, as lid sheet (113) winds up around the wheel (516) the effective winding surface increases to an ideal diameter and on further winding up further increases to a too great diameter for uniform indexing.

6. A medicament dispenser according to claim 5, wherein the ideal diameter of the effective winding surface corresponds essentially to the point at which half of the lid sheet (113) is wound up on the wheel (516).

7. A medicament dispenser according to any of claims 1 to 6, wherein said indexing means comprise a rotatable index wheel (506) having recesses therein, said index wheel being engageable with a medicament carrier (101) in use with said medicament dispenser such that said recesses each receive a respective pocket (105,107,109) of the base sheet of a medicament carrier in use with said medicament dispenser.

8. A medicament dispenser according to any of claims 1 to 7, further comprising an indexing lever (520) for actuating said dispenser wherein said indexing lever is interconnected with said indexing means (506) and/or said lid driving means.

9. A medicament dispenser according to any of claims 1 to 8, additionally comprising a medicament carrier (101) having a plurality of pockets (105,107,109) containing medicament.

10. A medicament dispenser according to claim 9, wherein the medicament is in powdered or solid form.

## Patentansprüche

1. Medikamentenspender zur Verwendung mit einem Medikamententräger (101), der eine Vielzahl von Taschen (105, 107, 109) aufweist, um ein Medikament zu enthalten, wobei die Taschen entlang der Länge von zwei ablösbaren Bögen (111, 113), die aneinander befestigt sind, beabstandet sind und zwischen ihnen definiert sind, der Spender einen inneren Abgabemechanismus zum Zugriff auf das innerhalb des Medikamententrägers enthaltene Medikament aufweist, wobei der Mechanismus umfasst,
a) eine Öffnungsstelle (508) zur Aufnahme einer Tasche des Medikamententrägers;
b) eine Ablöse-Einrichtung, die positioniert ist um einen Grundbogen (111) und einen Deckbogen (113) von einer Tasche in Eingriff zu nehmen, die in der Öffnungsstelle (508) aufgenommen wurde, zum voneinander Lösen eines derartigen Grundbogens (111) und Deckbogens (113), um eine derartige Tasche zu öffnen, wobei die Ablöse-Einrichtung eine Deckel-Antriebseinrichtung zum Auseinanderziehen eines Deckbogens (113) und eines Grundbogens (111) von einer Tasche, die an der Öffnungsstation (508) aufgenommen wurde, umfasst, wobei die Deckel-Antriebseinrichtung ein Rad (516) mit einem festen Durchmesser umfasst, auf dem der Deckbogen (113) gewickelt ist, wobei das Rad (516) eine effektive Wicklungsoberfläche aufweist, deren Durchmesser zunimmt, je mehr Deckbogen um das Rad (516) gewickelt wird,
c) einen Auslass, der positioniert ist, um sich mit einer geöffneten Tasche in Verbindung zu befinden, durch die ein Nutzer ein Medikament aus einer derartigen geöffneten Tasche entnehmen kann; und
d) eine Indizierungseinrichtung (506) zum Indizieren, in Verbindung mit dem Auslass, der Taschen eines mit dem Medikamentenspender verwendeten Medikamententrägers, wobei die Indizierungseinrichtung mit der Deckel-Antriebseinrichtung derart verbunden ist, dass eine Bewegung von einer mit der Bewegung von der anderen korreliert,
außerdem eine Kompensationseinrichtung umfassend, um jegliche Zunahme des Durchmessers von der effektiven Wicklungsoberfläche während einer Verwendung des Spenders zu kompensieren und um **dadurch** sicherzustellen, dass der Medikamententräger auf jede Betätigung des Abgabemechanismus hin gleichmäßig indiziert wird, **dadurch gekennzeichnet, dass**
die Kompensationseinrichtung eine Verdrehungsfeder (522) umfasst, die an dem Rad (516) positioniert ist.

2. Medikamentenspender nach Anspruch 1, bei dem das Rad (516) eine Nabenform aufweist, die derart geformt ist, dass sie die Verdrehungsfeder (522) aufnimmt.

3. Medikamentenspender nach einem der Ansprüche 1 bis 2, bei dem die anfängliche effektive Wicklungsoberfläche derart ausgewählt ist, dass sie anfänglich ein gleichmäßiges Indizieren des Medikamententrägers vorsieht.

4. Medikamentenspender nach einem der Ansprüche 1 bis 2, bei dem die anfängliche effektive Wicklungsoberfläche derart ausgewählt ist, dass sie anfänglich ein ungleichmäßiges Indizieren des Medikamententrägers vorsieht.

5. Medikamentenspender nach Anspruch 4, bei dem der Durchmesser der anfänglichen effektiven Wicklungsoberfläche kleiner als erforderlich ist für ein gleichmäßiges Indizieren des Medikamententrägers, und wobei bei der Verwendung, da sich der Deckbogen (113) um das Rad (516) aufwickelt, die effektive Wicklungsoberfläche auf einen Idealdurchmesser zunimmt, und bei einem weiteren Aufwickeln ferner auf einen zu großen Durchmesser für ein gleichmäßiges Indizieren zunimmt.

6. Medikamentenspender nach Anspruch 5, bei dem der Idealdurchmesser der effektiven Wicklungsoberfläche im Wesentlichen einem Punkt entspricht, an dem die Hälfte des Deckbogens (113) auf das Rad (516) aufgewickelt ist.

7. Medikamentenspender nach einem der Ansprüche 1 bis 6, bei dem die Indizierungseinrichtung ein drehbares Index-Rad (506) mit Aussparungen darin aufweist, wobei das Index-Rad mit einem Medikamententräger (101), der mit dem Medikamentenspender verwendet wird, in Eingriff bringbar ist, derart, dass jede der Aussparungen eine jeweilige Tasche (105, 107, 109) des Grundbogens eines mit dem Medikamentenspender verwendeten Medikamententrägers empfängt.

8. Medikamentenspender nach einem der Ansprüche 1 bis 7, ferner mit einem Index-Hebel (520) zum Betätigen des Spenders, wobei der Index-Hebel mit der Indizierungseinrichtung (506) und/oder der Deckel-Antriebseinrichtung verbunden ist.

9. Medikamentenspender nach einem der Ansprüche 1 bis 8, zusätzlich mit einem Medikamententräger (101), der eine Vielzahl von Taschen (105, 107, 109) aufweist, die ein Medikament enthalten.

10. Medikamentenspender nach Anspruch 9, bei dem das Medikament in Pulverform oder fester Form vorliegt.

## Revendications

1. Distributeur de médicaments à utiliser avec un élément porteur de médicaments (101) comportant une pluralité de poches (105, 107, 109) contenant du médicament, dans lequel lesdites poches sont mutuellement espacées sur la longueur de deux feuilles détachables (111, 113) fixées l'une à l'autre, et définies entre ces dernières, ledit distributeur comprenant un mécanisme de distribution intérieur pour accéder audit médicament contenu à l'intérieur dudit élément porteur de médicaments, ledit mécanisme comprenant :
a) une station d'ouverture (508) pour recevoir une poche dudit élément porteur de médicaments ;
b) des moyens de décollement positionnés de manière à venir en prise avec une feuille de base (111) et une feuille de recouvrement (113) d'une poche ayant été réceptionnée dans ladite station d'ouverture (508) afin de séparer par décollement lesdites feuilles de base (111) et de recouvrement (113), pour ouvrir ladite poche, lesdits moyens de décollement comprenant des moyens d'entraînement de la partie de recouvrement pour séparer une feuille de recouvrement (113) d'une feuille de base (111) d'une poche ayant été réceptionnée à l'endroit de ladite station d'ouverture (508), lesdits moyens d'entraînement de la partie de recouvrement comprenant une roue de diamètre fixe (516) sur laquelle est enroulée ladite feuille de recouvrement (113), ladite roue (516) présentant une surface d'enroulement effective, le diamètre de cette dernière allant en augmentant au fur et à mesure que la feuille de recouvrement est enroulée autour de ladite roue (516).
c) un orifice de sortie, positionné de manière à être en communication avec une poche ouverte, à travers lequel un utilisateur peut retirer un médicament depuis ladite poche ouverte ; et
d) des moyens d'indexage, ou d'alignement, (506) pour aligner en communication avec lesdits orifices de sortie les poches d'un élément porteur de médicaments utilisé avec ledit distributeur de médicaments, lesdits moyens d'alignement étant interconnectés avec lesdits moyens d'entraînement de partie de recouvrement de telle manière que le mouvement de l'un soit corrélé au mouvement de l'autre;
comprenant en outre des moyens de compensation pour compenser toute augmentation de diamètre de ladite surface d'enroulement effective pendant l'utilisation du distributeur et assurer ainsi que ledit élément porteur de médicaments est aligné de façon uniforme lors de chaque manoeuvre dudit mécanisme de distribution, **caractérisés en ce que** les moyens de compensation comprennent un ressort de torsion (522) positionné à l'endroit de la roue (516).

2. Distributeur de médicaments selon la revendication 1, dans lequel la roue (516) est en forme de moyeu configuré de telle manière à pouvoir recevoir ledit ressort de torsion (522).

3. Distributeur de médicaments selon l'une quelconque des revendications 1 à 2, dans lequel la surface d'enroulement effective initiale est choisie de manière à procurer initialement un alignement uniforme de l'élément porteur de médicaments.

4. Distributeur de médicaments selon l'une quelconque des revendications 1 à 2, dans lequel la surface d'enroulement effective initiale est choisie de manière à procurer initialement un alignement non uniforme de l'élément porteur de médicaments.

5. Distributeur de médicaments selon la revendication 4, dans lequel le diamètre de la surface d'enroulement effective initial est inférieur à celui requis pour obtenir un alignement uniforme de l'élément porteur de médicaments et, en cours d'utilisation, au fur et à mesure que la feuille de recouvrement (113) s'enroule autour de la surface roue (516), la surface d'enroulement effective augmente jusqu'à atteindre un diamètre idéal et, en cas de poursuite de l'enroulement, augmente encore pour atteindre un diamètre trop important pour obtenir un alignement uniforme.

6. Distributeur de médicaments selon la revendication 5, dans lequel le diamètre idéal de la surface d'enroulement effective correspond sensiblement au point auquel la moitié de la feuille de recouvrement (113) est enroulée sur la roue (516).

7. Distributeur de médicaments selon l'une quelconque des revendications 1 à 6, dans lequel lesdits moyens d'alignement comprennent une roue d'indexage, ou d'alignement, (506) comportant des encoches, ladite roue d'alignement pouvant être mise en prise avec un élément porteur de médicaments (101) utilisé avec ledit distributeur de médicaments de telle manière que lesdites encoches reçoivent chacune une poche respective (105, 107, 109) de la feuille de base d'un élément porteur de médicaments utilisé avec ledit distributeur de médicaments.

8. Distributeur de médicaments selon l'une quelconque des revendications 1 à 7, comprenant en outre un levier d'indexage, ou d'alignement, (520) pour actionner ledit distributeur dans lequel ledit levier d'alignement est interconnecté aux dits moyens d'actionnement (506) et/ou aux dits moyens d'entraînement de feuille de recouvrement.

9. Distributeur de médicaments selon l'une quelconque des revendications 1 à 8, comprenant en outre un élément porteur de médicaments (101) comportant une pluralité de poches (105, 107, 109) contenant du médicament.

10. Distributeur de médicaments selon la revendication 9, dans lequel le médicament est sous forme pulvérulente ou solide.
